# EUROPEAN PATENT SPECIFICATION

(11) **EP 2 218 404 B1**
(45) Date of publication and mention of the grant of the patent: **05.09.2018**
(21) Application number: 09425051.1
(22) Date of filing: 13.02.2009
(51) Int. Cl.: A61B 17/02, A61B 17/34, A61B 17/30

(54) **A surgical lift device to assist in surgical access through skin, tissue and organs**
Chirurgische Hebevorrichtung zur Unterstützung des chirurgischen Zugangs durch Haut, Gewebe und Organe
Dispositif de levage chirurgical pour faciliter l'accès chirurgical à travers la peau, les tissus et les organes

(43) Date of publication of application: 18.08.2010
(73) Proprietor: Fondazione Istituto Italiano Di Tecnologia, 16163 Genova (IT)
(72) Inventor: Caldwell, Darwin, I-16010 Serra Ricco' (Genova) (IT); Davies, Brian, W4 3 DR London (GB)
(74) Representative: Deambrogi, Edgardo

(56) References cited:
- GB-A- 2 140 695
- US-A- 6 042 539
- US-A1- 2003 036 677

## Description

The present invention relates to a surgical lift device, according to the preamble of claim 1.

In order to assist in access to organs through the skin, for procedures such as minimally invasive surgery, a process of insufflation is often used in which a pressurised gas is passed through the skin and attached fat layers, in order to allow tools such as those in laparoscopic surgery, to have better access to organs and tissue inside the domed region that is then formed. This procedure is often performed to access the inside of the abdomen, but may also be undertaken to access any other organs, such as lungs or heart. The gas is normally inserted through a trocar which must be sealed in order to prevent the loss of gas. If this loss should happen during surgery, the procedure has to be abandoned or delayed until the pressure is restored.

A further disadvantage of insufflation is that the pressurised gas can be absorbed, causing the patient pain and bloating for some time subsequently. A further disadvantage of insufflation is that the instruments used in surgery must pass through the sealed trocar, leading to a considerable increase in friction between the trocar and the tool for in/out and axial rotation motions, so that much of the sense of touch of the tool against internal tissue is lost. Also, since the skin is tightened like the surface of a drum, any attempt to pivot the tools in pitch and yaw relative to the skin will meet considerable resistance, further degrading any sense of "feel" of tools against internal tissue. As a consequence, the surgeon has to rely primarily on vision to judge any tool/tissue contact and observe any resulting tissue compression in order to judge the magnitude of tool contact forces.

An alternative to insufflation has also been used in which hooks are placed through the skin and attached to cords and levers to lift the skin and fatty tissue away from underlying organs. However, this has not been popular due to the trauma caused to the skin and also the need to provide the hooks with an overhead support which can impede the surgical access.

EP 0 672 385 discloses a surgical lift device of the type defined at the beginning of the description. This device utilizes a suction member for gripping the external skin surface of a human body wall. This removes the need for the pressurised gas inside the human body, avoiding the pain caused by the insufflation process. However, even this device suffers from some drawbacks. In particular, it includes a lifting member for lifting the suction member, which may interfere with other devices or operators' movements during surgical interventions.

GB 2 140 695 discloses a device for use in performing examinations and surgical interventions within the abdominal cavity of a patient, comprising a cowling which is applicable to the abdominal integument of the patient and which is provided with an upper temporarily closable opening, whereby a negative pressure generated within the cowling raises the abdominal integument. Instruments may be inserted through the opening and via the abdominal integument into the abdominal cavity without damaging any internal organs and vessels which were initially in contact with the inner side of the abdominal integument.

According to the present invention, it is provided a surgical lifting device of the type defined at the beginning, having the features claimed in claim 1. Due to the fact that the domed structure determines the lifting of the human body wall, the surgical lift device does not need any separate lifting member. Therefore, any interference situation during surgical interventions is avoided. Furthermore, the low friction port device allows tools to easily pass through and allows the contact forces to be judged more readily, either through direct contact of tool on tissue or with the aid of additional force sensing enhancement.

Preferably, the domed structure is made of a material which is transparent to visible light.

In accordance with a preferred embodiment, the suction member comprises a flexible porous membrane which extends over the entire base side of the suction member.

According to a further embodiment, the suction member comprises an inner rigid porous membrane.

According to a further preferred embodiment, at least one, and preferably a plurality of apertures are formed through the suction member in order to give access to said external skin surface, said apertures being formed in such a way as to ensure the external skin surface may be sealed to the suction member in the nearby regions around said apertures.

According to an embodiment, the suction member is provided with at least one, and preferably a plurality of flexible tubular walls which respectively surround said apertures and extend from the wall of the domed structure to the base side thereof, said tubular walls being non-permeable to air and having respective terminal portions formed in such a way as to be sealed to said external skin surface. Preferably, the tubular walls are preformed so as to fold like a concertina during application of negative pressure.

According to an alternative embodiment, the suction member is provided with a non-permeable sheet which covers the inner side of the domed structure so that the apertures are covered, said non-permeable sheet being perforable in use for permitting tool insertion.

In accordance with a further embodiment, the low friction entry port device is integrally formed in the dome structure at one of the apertures thereof.

In accordance to an alternative embodiment the low friction entry port device comprises a support plate to be removably placed over one of the apertures of said suction member in such a way as to rest on the edge of such aperture, and the low friction port fixture is provided on said support plate.

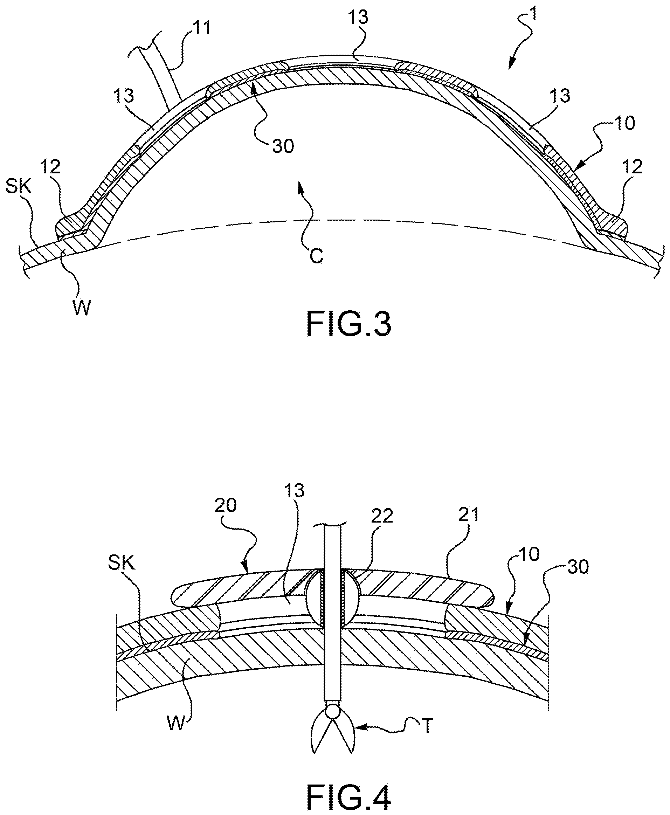

Some preferred, but non-limiting, embodiments of the invention will now be described, with reference to the attached drawings, in which Fig. 4 discloses an embodiment of the invention and the other figures disclose further embodiments of this disclosure usefull for the understanding of the invention:
- Figure 1 is a plan view sketch of a surgical lift device;
- Figure 2 is a sectional view of the device of Fig. 1 in a rest position, taken along the line II-II;
- Figure 3 is a sectional view sketch of the device of Fig. 1 in an operating position, taken along the line II-II;
- Figure 4 in an enlarged view of an aperture of the device of Fig. 1, equipped with a laparoscopic tool;
- Figure 5 is a sectional view sketch of the device of Fig. 1 applied over breast; and
- Figure 6 is a sectional view of another embodiment of a surgical lift device.

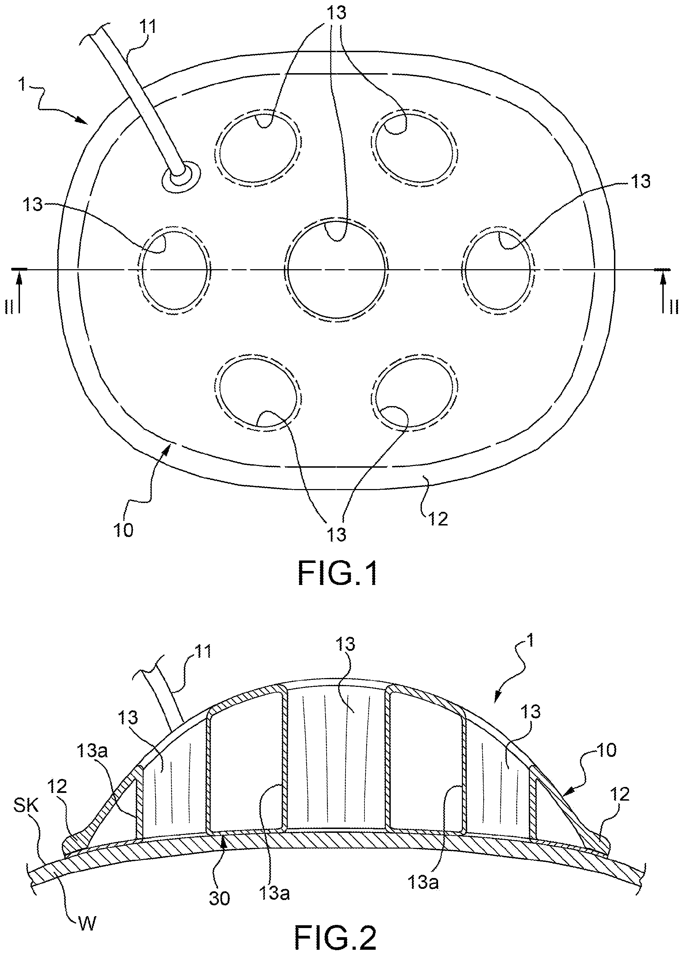

With reference to Figures 1 to 5, there is shown a surgical lift device, generally indicated with 1.

Surgical lift device 1 comprises a suction member 10 having a domed structure suitable for being placed on an external skin surface SK of a human body wall W, for example the skin surface of the abdominal wall. The suction member 10 is designed in such a way as to be sealed to the skin surface SK. Preferably, the material of the domed structure is transparent to visible light.

The suction member 10 is connectable to a vacuum source (not shown) through at least one vacuum line 11 for applying a negative pressure to the gap between skin surface SK and the domed structure. As shown in figure 3, this negative pressure shall be of a magnitude sufficient to lift the body wall W (i.e. skin together with attached fatty tissue) to conform to the shape of the dome structure leaving a cavity C on the back side of the body wall (the dashed line in Figure 3 schematically indicates the border of the area occupied by the internal organs). This removes the need for the pressurised gas inside the abdomen, avoiding the pain previously caused.

The domed structure of the suction member 10 is load-bearing (in other words, rigid or semi-rigid), i.e. it does not collapse under the action of the negative pressure, or it collapses to a limited extent with respect to the lifting movement of the human body wall. Therefore, the domed structure determines the lifting of said human body wall during application of negative pressure. This feature removes the need for a separate lifting member, such as that provided in EP 0 672 385.

The domed structure can be provided in a range of shapes and sizes to suit different sized patients or different areas of the body, such as the breast. The edges 12 of the domed structure can be flexible to enhance sealing of the region of negative pressure to the skin surface SK.

At least one, and preferably a plurality of apertures 13 are formed through the suction member 10 in order to give access to the skin surface SK. These apertures 13 are formed in such a way as to ensure the skin surface SK is sealed to the suction member 10 in the nearby regions around the apertures 13. As shown in Fig. 2, this may be obtained by forming flexible tubular walls 13a which extend from the wall of the domed structure to the base side thereof. These tubular walls 13a are non-permeable to air. At the base side of the domed structure, the terminal portions of these flexible tubular walls are formed in such a way as to be sealed to the external skin surface SK. The tubular walls 13a are formed so as that they are less likely to cover the apertures 13 as they collapse upon vacuum application. For example, the tubular walls 13a may be preformed so as to fold like a concertina.

The areas of the apertures 13 can be used to insert endoscopes and tools without restriction from skin tension. If a trocar were used in these areas, it would not need to be gas sealed and so simple low-friction features could be used in the trocar to allow tools to pass through and allow the contact forces to be judged more readily, either through direct contact of tool on tissue or with the aid of additional force sensing enhancement. Tool and endoscope in/out and axial rotation motions may be further enhanced by the use of slippery coatings or low friction devices such as recirculating ball-races or screws. Alternatively, if a trocar were not used, a specially designed access port can allow low-force contact with tissue. An additional benefit from the device is that it can facilitate low friction pitch and yaw motions of the laparoscopic tools and endoscopes, without the restriction previously caused by insufflation and stretched skin in the region. This can be enhanced by utilising low friction pivots at the access port or trocar device, allowing an enhanced sense of feel when pivoting the tools in order to contact tissue. A particular embodiment of the invention is shown in Figure 4. This embodiment uses a region of the domed structure of the suction member 10 as a support for a low friction entry port device 20. This low friction entry port device 20 comprises a support plate 21 to be placed over one of the apertures 13 of the suction member 10 in such a way as to rest on the edge of such aperture. A low friction port fixture 22, such as a low friction pivot, is provided on the support plate 21 for inserting a tool T. In this way, the tool T may be operated through port fixture 22 and aperture 13. According to a further embodiment (not shown), the low friction entry port device may be integrated in the domed structure at one of the apertures 13 thereof. It is to be understood that the port device shown in Figure 4 is only an example; many other kinds of devices are available which may be coupled to the domed structure of the present invention. In any case the tools may either be moved manually or by actuators and control systems that form part of a robotic manipulator.

As shown in the drawings, the suction member 10 may be provided with a flexible porous membrane 30 which extends over the entire base side of the suction member 10. In this case, the negative pressure may be applied between the membrane 30 and the domed structure to facilitate sealing whilst minimising skin trauma. Alternatively or in combination, a rigid porous membrane (not shown) may be used inside the domed structure. In this case the negative pressure may be placed between the domed structure and the rigid membrane. According to a further alternative (not shown), the domed structure is directly placed to the skin, without intermediate membrane. In this case, the negative pressure is directly applied in the gap between the domed structure and the skin surface. Both domed structure and membrane may be transparent to enhance vision of the surgical site. The negative pressure is determined so as to be sufficient to lift the skin to the dome, whilst not being too great to cause the capillaries to burst.

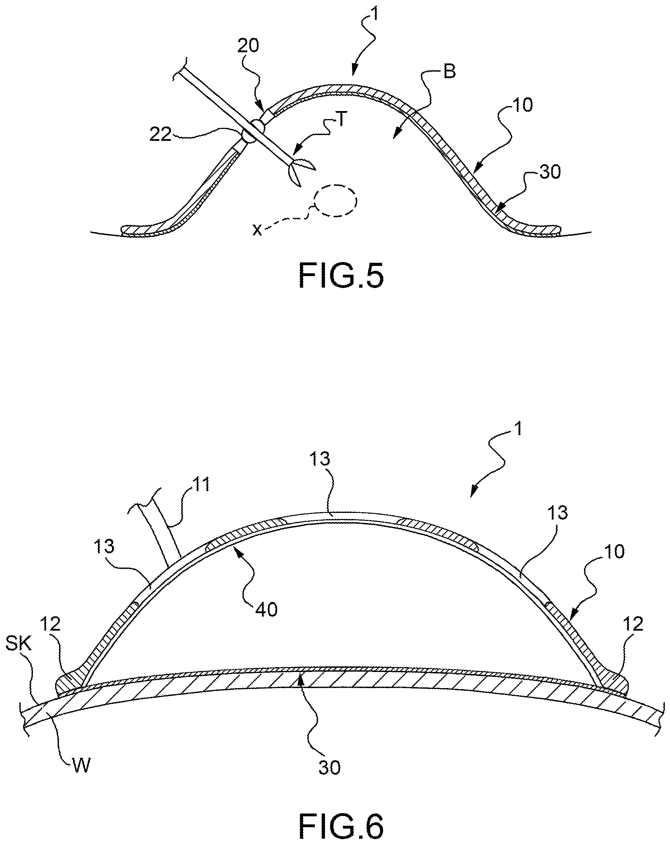

A further benefit of the domed negative pressure structure is that it can be used to condition the tissue or organ to form a constant shape defined by the dome without changing shape, such as due to gravitational effect during a change of pose. An example of this is in the breast diagnostic and surgery, as shown in Figure 5. In this Figure, B indicates the breast zone, while X indicates the position of a tumour. It is known that in breast diagnostic images may be taken prone whilst a surgical operation may be performed supine. This can lead to a considerable difference in shape of the organ due to gravitational effects when changing pose. The use of the proposed device to condition the breast to a constant form can ensure a consistent shape between imaging and intervention, irrespective of pose.

Figure 6 shows another embodiment of a surgical lift device. Elements corresponding to those of Figs. 1 to 5 are identified by like reference numerals. The embodiment of Fig. 6 represents an alternative viable and cost-effective means of avoiding air being sucked through apertures 13 when applying negative pressure. This embodiment does not have the tubular walls 13a, but is provided with a thin transparent plastic non-permeable sheet 40 which covers the inner side of the domed structure so that the apertures 13 are covered. Once a position corresponding to that of Fig. 3 is attained, and the porous membrane 30 covers the inner side of the domed structure sealing around the edge of the apertures 13, the sheet 40 can be perforated at 13, permitting tools and telescopes to be passed through the holes and through the skin and tissue.

It is to be understood that the embodiments shown in the Figures are only examples. There are a number of other possible means of sealing the apertures 13 which are available to the person skilled in the art. Some examples comprise sliding covers, a separate external dome to seal the domed structure and apertures until position in Fig. 3 is achieved, partially excised discs, and so on. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A surgical lift device (1), comprising a suction member (10) for detachably contacting an external skin surface (SK) of a human body wall (W) and holding said external skin surface by means of application of negative pressure between said suction member and said external skin surface, said suction member having a gripping force sufficient to permit lifting of said human body wall to an elevated position and to hold said human body wall in said elevated position, wherein said suction member has a load-bearing domed structure which determines the lifting of said human body wall during application of negative pressure, wherein said surgical lift device further comprises an entry port device (20) arranged on the domed structure; **characterised in that** said entry port device (20) is a low friction entry port device (20), said low friction entry port device comprising a low friction pivot (22) for inserting a tool (T),
at least one, and preferably a plurality of apertures (13) being formed through the suction member (10) in order to give access to said external skin surface, said apertures being formed in such a way as to ensure the external skin surface (SK) may be sealed to the suction member (10) in the nearby regions around said apertures,
wherein said low friction entry port device comprises a support plate (21) to be removably placed over one of the apertures (13) of said suction member in such a way as to rest on the edge of such aperture, and said low friction pivot (22) is provided on said support plate.

2. A surgical lift device according to claim 1, wherein said domed structure is made of a material which is transparent to visible light.

3. A surgical lift device according to Claim 1 or 2, wherein said suction member comprises a flexible porous membrane (30) which extends over the entire base side of the suction member.

4. A surgical lift device according to any one of Claims 1 to 3, wherein said suction member comprises an inner rigid porous membrane.

5. A surgical lift device according to Claim 1, wherein said suction member is provided with at least one, and preferably a plurality of flexible tubular walls (13a) which respectively surround said apertures and extend from the wall of the domed structure to the base side thereof, said tubular walls being non-permeable to air and having respective terminal portions formed in such a way as to be sealed to said external skin surface.

6. A surgical lift device according to Claim 5, wherein said tubular walls are preformed so as to fold like a concertina during application of negative pressure.

7. A surgical lift device according to Claim 1, wherein said suction member is provided with a non-permeable sheet (40) which covers the inner side of the domed structure so that the apertures (13) are covered, said non-permeable sheet being perforable in use for permitting tool insertion.

## Patentansprüche

1. Chirurgische Hebevorrichtung (1) mit einem Saugbauteil (10) zum lösbaren Kontaktieren einer äußeren Hautoberfläche (SK) einer menschlichen Körperwand (W) und Halten der äußeren Hautoberfläche mittels Aufbringen eines Unterdrucks zwischen dem Saugbauteil und der äußeren Hautoberfläche, wobei das Saugbauteil eine Haltekraft aufweist, die ausreicht zum Ermöglichen eines Anhebens der menschlichen Körperwand zu einer angehobenen Position und zum Halten der menschlichen Körperwand in der angehobenen Position, wobei das Saugbauteil eine lasttragende gewölbte Struktur aufweist, die das Anheben der menschlichen Körperwand während des Aufbringens des Unterdrucks bestimmt, wobei die chirurgische Hebevorrichtung ferner eine Eingangsöffnungsvorrichtung (20), die an der gewölbten Struktur angeordnet ist, aufweist; **dadurch gekennzeichnet, dass** die Eingangsöffnungsvorrichtung (20) eine Eingangsöffnungsvorrichtung (20) mit niedriger Reibung ist, wobei die Eingangsöffnungsvorrichtung mit niedriger Reibung einen Drehpunkt (22) mit niedriger Reibung zum Einführen eines Werkzeugs (T) aufweist,
mindestens eine, bevorzugt mehrere Öffnungen (13) zum Zugänglichmachen der äußeren Hautoberfläche durch das Saugbauteil (10) ausgebildet sind, wobei die Öffnungen derart ausgebildet sind, dass sichergestellt ist, dass die äußere Hautoberfläche (SK) in den Regionen in der Nähe der Öffnungen gegenüber dem Saugbauteil (10) abgedichtet werden kann,
bei der die Eingangsöffnungsvorrichtung mit niedriger Reibung eine Tragplatte (21) aufweist, die abnehmbar über einer der Öffnungen (13) des Saugbauteils zu platzieren ist, so dass sie an dem Rand einer solchen Öffnung aufliegt, und der Drehpunkt (22) mit niedriger Reibung an der Tragplatte vorgesehen ist.

2. Chirurgische Hebevorrichtung nach Anspruch 1, bei der die gewölbte Struktur aus einem Material besteht, das für sichtbares Licht transparent ist.

3. Chirurgische Hebevorrichtung nach Anspruch 1 oder 2, bei der das Saugbauteil eine flexible poröse Membran (30) aufweist, die sich über die gesamte Basisseite des Saugbauteils erstreckt.

4. Chirurgische Hebevorrichtung nach einem der Ansprüche 1 bis 3, bei der das Saugbauteil eine innere starre poröse Membran aufweist.

5. Chirurgische Hebevorrichtung nach Anspruch 1, bei der das Saugbauteil mit mindestens einer, bevorzugt mehreren flexiblen röhrenförmigen Wänden (13a) versehen ist, die jeweils die Öffnungen umgeben und sich von der Wand der gewölbten Struktur zu der Basisseite derselben erstrecken, wobei die röhrenförmigen Wände nicht durchlässig für Luft sind und jeweilige Endabschnitte aufweisen, die so ausgebildet sind, dass sie mit der äußeren Hautoberfläche dichten.

6. Chirurgische Hebevorrichtung nach Anspruch 5, bei der die röhrenförmigen Wände so vorgebildet sind, dass sie während eines Aufbringens des Unterdrucks wie eine Ziehharmonika gefaltet werden.

7. Chirurgische Hebevorrichtung nach Anspruch 1, bei der das Saugbauteil mit einer nicht durchlässigen Lage (40) versehen ist, die die Innenseite der gewölbten Struktur bedeckt, so dass die Öffnungen (13) bedeckt sind, wobei die nicht durchlässige Lage während einer Verwendung zum Erlauben eines Einführens eines Werkzeugs perforiert werden kann.

## Revendications

1. Dispositif de levage chirurgical (1), comprenant un élément d'aspiration (10) pour entrer en contact de façon amovible avec une surface extérieure de peau (SK) d'une paroi de corps humain (W) et maintenir ladite surface extérieure de peau au moyen d'une application de pression négative entre ledit élément d'aspiration et ladite surface extérieure de peau, ledit élément d'aspiration ayant une force de préhension suffisante pour permettre le levage de ladite paroi de corps humain à une position élevée et pour maintenir ladite paroi de corps humain dans ladite position élevée, dans lequel ledit élément d'aspiration a une structure porteuse en forme de dôme qui détermine le levage de ladite paroi de corps humain durant l'application de pression négative, dans lequel ledit dispositif de levage chirurgical comprend en outre un dispositif d'orifice d'entrée (20) disposé sur la structure en forme de dôme ; **caractérisé en ce que** ledit dispositif d'orifice d'entrée (20) est un dispositif d'orifice d'entrée(20) à faible frottement, ledit dispositif d'entrée à faible frottement comprenant un pivot à faible frottement (22) pour l'insertion d'un outil (T),
au moins une, et de préférence une pluralité d'ouvertures (13) étant formées à travers l'élément d'aspiration (10) pour permettre l'accès à ladite surface extérieure de peau, lesdites ouvertures étant formées de manière à assurer que la surface extérieure de peau (SK) puisse être scellée à l'élément d'aspiration (10) dans les régions proches autour desdites ouvertures,
dans lequel ledit dispositif d'orifice d'entrée à faible frottement comprend une plaque de support (21) à placer de manière amovible pardessus l'une des ouvertures (13) dudit élément d'aspiration de manière à ce qu'elle repose sur le bord de cette ouverture, et ledit pivot à faible frottement (22) est fourni sur ladite plaque de support.

2. Dispositif de levage chirurgical selon la revendication 1, dans lequel ladite structure en forme de dôme est en un matériau qui est transparent à la lumière visible.

3. Dispositif de levage chirurgical selon la revendication 1 ou 2, dans lequel ledit élément d'aspiration comprend une membrane poreuse souple (30) qui s'étend sur la totalité du côté base de l'élément d'aspiration.

4. Dispositif de levage chirurgical selon l'une quelconque des revendications 1 à 3, dans lequel ledit élément d'aspiration comprend une membrane poreuse rigide interne.

5. Dispositif de levage chirurgical selon la revendication 1, dans lequel ledit élément d'aspiration est doté d'au moins une, et de préférence d'une pluralité de parois tubulaires souples (13a) qui entourent respectivement lesdites ouvertures et s'étendent de la paroi de la structure en forme de dôme jusqu'à son côté base, lesdites parois tubulaires étant non perméables à l'air et ayant des parties terminales respectives formées de manière à être scellées à ladite surface extérieure de peau.

6. Dispositif de levage chirurgical selon la revendication 5, dans lequel lesdites parois tubulaires sont préformées de façon à se plier comme un accordéon durant l'application de pression négative.

7. Dispositif de levage chirurgical selon la revendication 1, dans lequel ledit élément d'aspiration est doté d'une feuille non perméable (40) qui couvre le côté intérieur de la structure en forme de dôme de manière à ce que les ouvertures (13) soient couvertes, ladite feuille non perméable étant perforable durant l'utilisation pour permettre l'insertion d'outils.
